Europäisches Patentamt

European Patent Office

Office européen des brevets

(19)

(11) Publication number: **0 333 691**
**A2**

# EUROPEAN PATENT APPLICATION

(21) Application number: **89850091.3**

(22) Date of filing: **16.03.89**

(51) Int. Cl.⁴: **C 12 N 15/00**
C 07 K 3/12, C 12 N 1/20
//(C12N1/20,C12R1:07,1:185,
1:44)

(30) Priority: **17.03.88 SE 8800981**

(43) Date of publication of application:
**20.09.89 Bulletin 89/38**

(84) Designated Contracting States:
**AT BE CH DE FR GB IT LI NL SE**

(71) Applicant: **CEMU BIOTEKNIK AB**
**Kungsgatan 46**
**S-753 21 Uppsala (SE)**

(72) Inventor: **Nygren, Per-Ake**
**Pilotgatan 22**
**S-12251 Enskede (SE)**

**Abrahmsen, Lars**
**Själagardsgatan 10**
**S-111 31 Stockholm (SE)**

**Uhlen, Mathias**
**Kvarnbogatan 30**
**S-752 39 Uppsala (SE)**

(74) Representative: **Henningsson, Gunnar et al**
**Bergling & Sundbergh AB Box 7645**
**S-103 94 Stockholm (SE)**

(54) **A recombinant fusion protein, its use and a recombinant vector.**

(57) A recombinant fusion protein comprising a desired protein, oligo- or polypeptide flanked by affinity fragments or moieties, each capable of selective bindning; recombinant vector, process for purifying and isolating a desired protein; and a host organism transferred by the recombinant vector.

Fig. 1

```
                                                              -5
                                                              A1
                                                              AAT TCT
                                                                  GA
                                                                  B1

1       7   13      19      25      31      37      43
MET ALA TYR ARG PRO SER GLU THR LEU CYS GLY GLY GLU LEU VAL ASP
        A2                              A3
ATG GCT TAC CGT CCG TCT GAA ACC CTG TGC GGT GGT GAA CTG GTT GAC
TAC CGA ATG GCA GGC AGA CTT TGG GAC ACG CCA CCA CTT GAC CAA CTG
                B2                              B3

49      55      61      67      73      79      85      91
THR LEU GLN PHE VAL CYS GLY ASP ARG GLY PHE TYR PHE SER ARG PRO
        A4                      A5                      A6
ACC CTG CAG TTC GTT TGC GGT GAT CGT GGT TTC TAC TTC TCT CGT CCA
TGG GAC GTC AAG CAA ACG CCA CTA GCA CCA AAG ATG AAG AGA GCA GGT
            B4                      B5

97      103     109     115     121     127     133     139
ALA SER ARG VAL SER ARG ARG SER ARG GLY ILE VAL GLU GLU CYS CYS
                A7                      A8
GCT TCT CGT GTT TCT CGT CGT TCT CGA GGT ATC GTT GAA GAA TGC TGT
CGA AGA GCA CAA AGA GCA GCA AGA GCT CCA TAG CAA CTT CTT ACG ACA
B6                              B7                      B8

145     151     157     163     169     175     181     187
PHE ARG SER CYS ASP LEU ALA LEU LEU GLU THR TYR CYS ALA THR PRO
        A9                      A10                         A11
TTT CGT TCT TGT GAC CTG GCT CTG CTG GAA ACC TAC TGC GCT ACC CCG
AAA GCA AGA ACA CTG GAC CGA GAC GAC CTT TGG ATG ACG CGA TGG GGA
                B9                      B10

193     199
ALA LYS SER GLU

GCG AAA TCT GAA TAA TA
CGC TTT AGA CTT ATT ATT CGA
            B11
```

EP 0 333 691 A2

## Description

## A recombinant fusion protein, its use and a recombinant vector

The present invention relates to recombinant fusion proteins, their use in preparing protein and polypeptide products with high purity and the invention also relates to recombinant vectors capable of replication in a host cell and a process for purifying and isolating a desired protein, oligo-or polypeptide. More specifically, the invention relates to a dual flanking affinity fusion protein, oligo- or polypeptide, in which a desired protein is fused between two protein, oligo- or polypeptide fragments capable of selective binding.

Gene fusion is a procedure wherein the coding sequence of two or more genes are spliced together to form a combined gene which on expression in a suitable host organism will produce a fusion product wherein the separate proteins or polypeptides code for by the respective genes are fused together into a single molecule.

A dual flanking affinity gene fusion, as defined here, is a gene fusion encoding a recombinant fusion protein which schematically can be denoted as:

A - X - B

where X is one or several desired proteins or polypeptides and A and B are two separate or identical oligo- or polypeptides capable of selective binding. The three parts are spliced together with gene fusion techniques as will be explained below.

The present invention provides means to facilitate immobilization and purification of proteins through a method based upon recombinant DNA technology which permits desired proteins and polypeptides to be produced with extreme purity. According to the invention this is achieved by utilizing the specific binding of the A and B fragments to specific ligands. The produced A - X - B fusion protein or polypeptide can easily be isolated with high efficiency by conventional affinity chromatography utilizing suitable ligands, selecti vely binding to A and B, respectively, immobilized to a suitable carrier. The purification may be achieved by a two-step procedure in which the fusion protein is affinity purified first by the A-binding carrier and then by the B-binding carrier or vice versa . The purified fusion protein is thus purified by selective binding parts from both the C-terminal and N-terminal side of the desired protein or polypeptide which ensures a product including the desired protein or polypeptide. Using this procedure it is thus possible to greatly facilitate the problems with proteolytic degradation of the recombinant protein, which yields the purified fusion protein lacking parts or the whole of the desired protein or polypeptide. Such degraded products are co-purified with conventional gene fusion techniques involving only one ligand (Nilsson et al. EMBOJ. 4, 1075-1080, 1984) and might cause considerable problems in the following purification steps.

The desired protein or polypeptide X can consist of one or several parts, either identical or different. Preferably these parts can be cleaved apart by a chemical or enzymatic method after purification of the full length fusion protein. This provides the advantage that the molar amounts of desired product can be increased per mole of recombinant fusion protein.

The specific binding fragments A and B can be any oligo- or polypeptide capable of selective binding to a suitable carrier. Examples of such fragments are staphylococcal protein A (Nilsson et al. EMBOJ. 4, 1075-1080, 1985), streptococcal protein G (Guss et al, EMBOJ. 4, 1567-1575, 1986) and the synthetic fragment Z (Nilsson et al, Prof.Eng. 1, 107,113, 1987) and derivatives thereof, all capable of selective binding to the ligand IgG. Other suitable fragments include the human serum albumin-binding fragment from streptococcal protein G (copending patent application 8800378-5), streptavidin from Streptomyces avidii and hen avidin both binding to biotin and biotin-analogues, fibronectin-binding fragments (carbohydrate binding fragments) etc. Also possible is oligopep tides containing one or several cysteines, thus selectively binding to thiol-containing matrices. The optimal choice of fragments A and B for a given desired protein or polypeptide depends on a number of factors, such as host bacterium, stability, folding, secretion, site-specific cleavage method etc.

A basic aspect of the present invention is thus the provision of a recombinant DNA cloning vehicle or vector comprising a DNA sequence coding for a desired protein or polypeptide operatively linked to a DNA sequence coding for A and B parts, such that said DNA sequences together code for an A - X - B fusion product. In order to be capable of transforming (which is also meant to include the case that the vector is a bacteriophage or a virus) a host organism to produce said fusion product, the vector in conventional manner further comprises a promoter for the combined fusion product coding DNA sequence. For purposes which will be further elucidated below said combined DNA sequence may comprise a sequence coding for appropriate cleavage sites between the DNA sequences coding for the desired protein and the A and B parts, respectively, such that earlier or both the A and B parts of the fusion molecule may be cleaved off as mentioned above.

By transforming a compatible host organism with said vector to permit expression of the above combined DNA sequence and culturing the host in a nutrient medium the corresponding A- X - B fused protein or polypeptide will be produced. Although bacterial hosts, such as strains of, for example, Escherichia, Bacillus and Staphylococcus, are preferred for the purposes of the invention, it is, of course, also within the scope thereof to use other hosts, such as yeasts and other fungi, plant cells and mammalian cells in culture, etc. The transformation of the hosts may be effected with well-known methods.

Due to the specific A and B binding fragments of the fusion molecule produced by the cultured host-organism the full-length fusion molecule can be very efficiently isolated from the cell culture by means of ligands immobilized to two suitable carriers. If the fusion product is secreted into the surrounding medium the binding to the first carrier may be performed directly from the medium. If, on the other hand, the fusion product remains within the cells the latter have to be ruptured before such binding can be effected. Rupture of the cell walls may be effected in conventional manner by, e.g., high pressure, ultrasonication, homogenization, shaking with glass-beads etc. In cases where the product is trapped within the periplasmic space between two cell membranes, as in gram-negative bacteria, an osmotic shock procedure may be used to release the product into the suspension medium. Any other treatment of the cultured cells or the growth medium prior to the affinity isolation of the fusion product is, of course, also within the scope of the invention.

In conventional manner the immobilization process may be preferred batch-wise with the ligand-coupled carrier slurried in a suitable medium or on a column of the activated carrier. Any conventional carrier material to which the ligands can be sufficiently coupled for the present purposes may be used. The methods for coupling or immobilizing ligand to such carrier materials is well-known and need not be described in any detail herein. It is possible to use adsorption of ligand to surfaces of microtiter wells as a means for coating.

Release or desorption of the fused protein or polypeptide which is bound to the ligand-carrier depend on the choice of ligand-binding. Usually, as in the case for albumin-binding fragments and protein A derived fragments this may be effected by conventional methods, such as lowering the pH, e.g. by means of acetic acid buffer (pH 2.7), treatment with high salt concentrations or chaotrophic ions, or by competitive elution using excess soluble HSA or IgG to displace the fusion protein or polypeptide from the HSA- or IgG-carrier adsorbent. The choice of desorption method should, of course, be made with regard to the particular desired protein or polypeptide, such that a desired activity thereof is not lost or considerably reduced thereby. From the resulting eluate the fusion protein or polypeptide may readily be isolated and, if desired, subjected to further purification steps, such as gel filtration, ion exchange etc.

The purified protein or polypeptide obtained may in itself be a valuable product as will be described below, and another aspect of the present invention is therefore the provision of a method of producing a highly purified fused protein or polypeptide product comprising the steps of transforming a compatible host with the above vector, culturing said host in a nutrient medium, isolating said fused protein or polypeptide from said host culture by selective binding thereof to a ligand-supporting carrier, and optionally releasing the fused protein or polypeptide from the carrier, as well as such an isolated fused product obtained thereby.

The A and B binding fragments of the fused protein or polypeptide may under certain conditions be cleaved off, the pure desired protein or polypeptide thereby being obtained. In another aspect the present invention therefore provides a method of producing a desired protein or polypeptide of high purity comprising the steps of transforming a compatible host with the above mentioned vector, culturing said host in a nutrient medium, isolating said fused protein or polypeptide from the cell culture by selective binding to one or two ligand-supporting carriers, and cleaving off the desired protein or polypeptide from the A and/or B parts of said fused protein or polypeptide, either directly from the carrier bound fusion product or after desorption thereof from the carrier.

A necessary condition to permit such cleavage of the fused protein or polypeptide is, of course, that it contains cleavage sites which may be recognized and cleaved by suitable means. Such a cleavage site may be an amino acid sequence recognizable by chemical or enzymatic means and located between the desired protein or polypeptide and the flanking affinity parts of the fused product to be produced. Such a specific amino acid sequence must not occur within the desired protein or polypeptide and preferably not in the binding part of the fusion product. Examples of enzymatic agents include proteases, such as factor Xa, which in some cases recognizes the amino acid sequence $NH_2$-Ile-Glu-Gly-Arg-COOH ; chymosin (rennin), which cleaves the Met-Phe bond; kallikrein B, which cleaves on the carboxyl side of Arg in X-Phe-Arg-Y; enterokinase, which recongizes the sequence X-$(Asp)_n$-Lys-Y, wherein $n=2$-4, and cleaves it on the carboxyl side of Lys; thrombin which cleaves at specific arginyl bonds. Examples of chemical agents include cyanogen bromide (CNBr), which cleaves after Met; hydroxylamine, which cleaves the Asn-Gly bond, formic acid, which in high concentration ($\sim 70\%$) specifically cleaves Asp-Pro.

As appears from the above a crucial part of the present invention is the provision of the recombinant DNA structure or vector comprising the combined gene coding for the present fusion protein or polypeptide and capable of transforming a host cell to permit expression thereof and production of the fusion product. The present invention is meant to encompass any such vector irrespective of how it has been obtained using, for example, various restriction enzyme cutting, ligating, transforming and screening techniques well-known in the art as well as any appropriate vector materials and host-organisms. Thus, the DNA sequence coding for the desired protein or polypeptide may be inserted into a suitable vector and the A and B fragments coding DNA sequence inserted subsequently, or vice versa: or the three DNA sequences may be introduced simultaneously into the vector. It is also possible to insert the respective DNA sequences in parts thereof into the vector. The special techniques for accomplishing such insertions and combinations with maintained correct reading frames, including the provision of suitable restriction sites therefore, are well-known per se in the art.

The invention also covers a recombinant DNA molecule comprising the recombinant DNA sequence as described above and fused thereof at DNA level a production gene. By this arrangement such molecule obtains the ability to express a fused protein in a suitable host. Such production gene may be that of a somatomedin, examples of which are: growth hormones or factors, such as hGH (Human Growth Hormone), IGF-I, IGF-II, NGF (Nerve Growth Factor), EGF (Epidermal Growth Factor) and PDGF (Platelet Derived Growth Factor). The production gene may also be one coding for an interfon, interleu-Kin-2, insulin, neuropeptide, gastrointestinal peptides, secretin tissue plasminogen activator (tPA) and active derivatives thereof, etc. The production gene may also code for a structural gene for an enzyme or parts thereof.

According to still another aspect of the invention there is provided a process for cleaving a fused protein expressed in a biological system by the recombinant DNA molecule as defined above.

Finally, the invention covers a plasmid vector comprising the recombinant DNA molecule as described above. The invention also extends to bacterial or eucaryotic cells harbouring the recombinant DNA molecule defined above. The molecule can be inserted in the chromosome of the cell but may also be contained in a plasmid vector.

The host cell is for example a Gram negative bacterium and is particularly constituted by an E.coli.

The invention will in the following be further illustrated by non-limiting examples with reference to the appended drawings wherein:

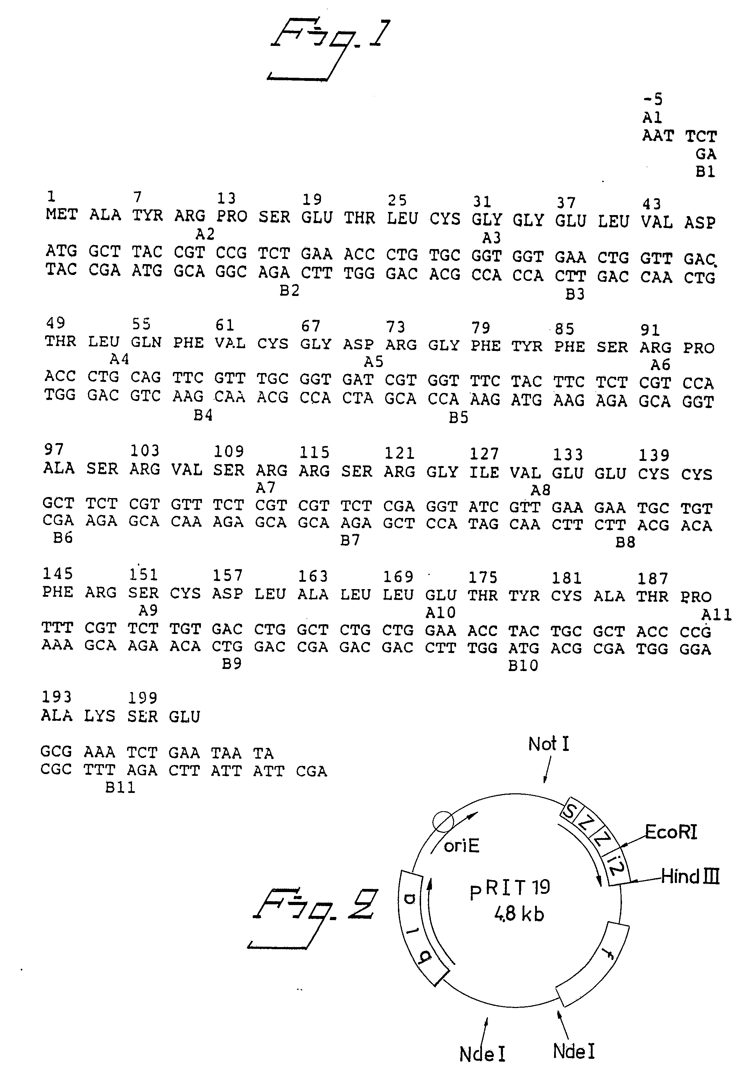

Fig. 1 shows the sequence of the synthetic human IGF-II gene before modification. The start for the 22 oligonucleotides used to assemble the gene is indicated (A1-A11 and B1-B11), as well as the deduced amino acid sequence. A methionine is positioned in front of the structural gene to enable cleavage of the fusion protein and double TAA codon is inserted as a translation stop signal. The gene is flanked by EcoRI and HindIII restriction sites.

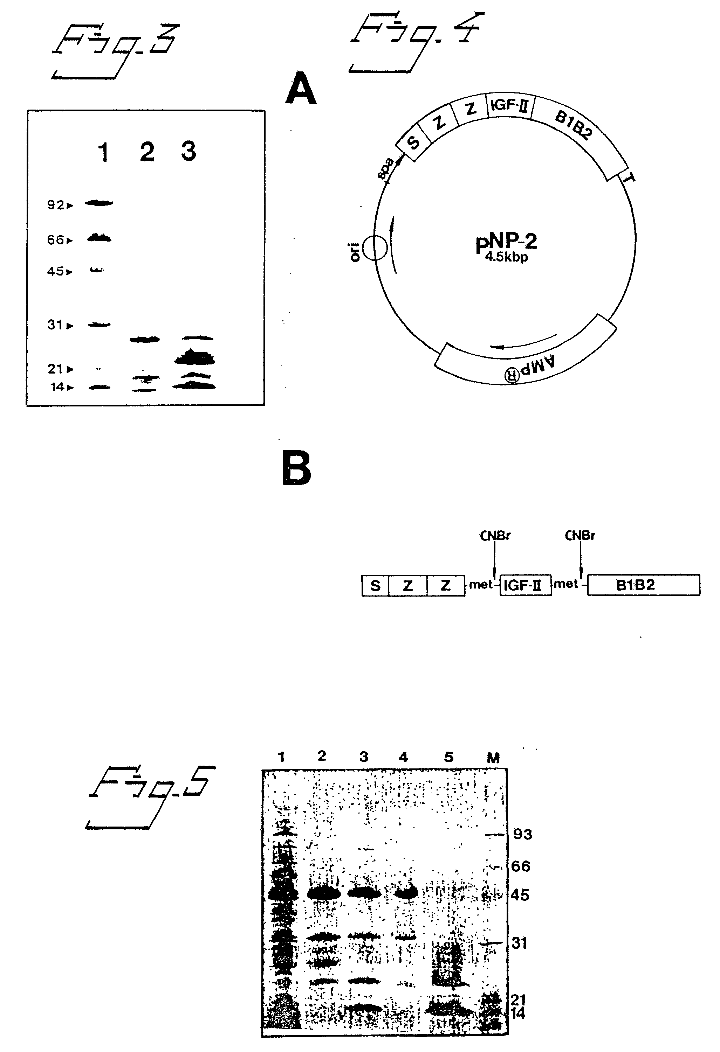

Fig. 2 is a schematic drawing of the expression vector encoding the human IGF-II fusion protein. Some relevant restriction sites are indicated. Boxes represent the genes coding for the signal sequence (S), synthetic IgG-binding region (Z), IGF-II (i2) and β-lactamase (bla). The origin of replication in E.coli (oriE) is also shown.

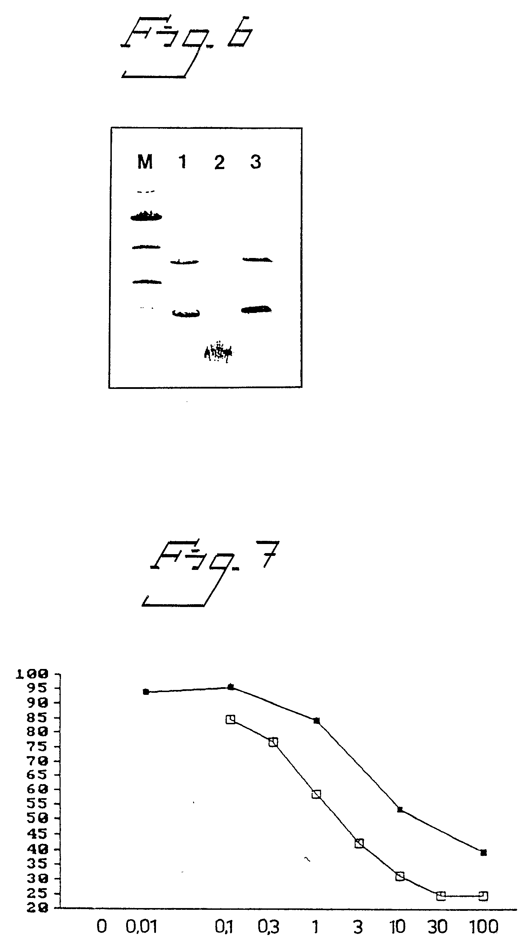

Fig. 3 shows the differential stability of ZZ IGF-I and ZZ IGF-II, respectively, produced in E.coli. Lane 1. Marker protein; lane 2. ZZ IGF-I, affinity purified on IgG-I Sepharose; lane 3. ZZ IGF-II purified on IgG Sepharose.

Fig. 4a shows a schematic drawing of the expression vector encoding the dual affinity fusion protein ZZ IGFII-BIB2. Boxes (not drawn to scale) represent the genes coding for the signal sequence (S), synthetic IgG-binding region (Z), human insulin growth factor II (IGF-II), albumin-binding part (BIB2) and β-laktamase (AMP).

Fig. 4b shows schematically the dual affinity construct with the IGF-II flanked N-terminally by the IgG-binding domains ZZ, and C-terminally by the albumin-binding domain. The two methionin-residues, positioned immediately before and after the IGF-II protein, provide sites for specific cleavage by CNBr, releasing free IGF-II.

Fig. 5 shows an analysis by SDS-PAGE of periplasmic proteins produced by E.coli HB101 cells containing the pNP-2 construct (Fig 4a) .Lane 1. total periplasmic content; lane 2. IgG-affinity purified proteins; lane 3. HSA-affinity purified proteins; lane 4. Proteins purified first by HSA-affinity and later also be IgG-affinity; lane 5. Proteins purified by HSA-affinity, but flow through in the following purification using IgG-affinity; lane 5. Marker proteins.

Fig. 6 shows an analysis by SDS-PAGE of purified fusion protein (Fig. 4b, except for the in vivo cleaved signal sequence) before and after treatment with CNBr; Lane 1. Fusion purified using both HSA- and IgG-affinity; lane 2. Purified fusion protein after treatment with CNBr (total); lane 4. Proteins from flow-through using a mixed IgG- and HSASepharo se column (pure IGF-II); lane 8. Proteins eluted from the mixed column.

Fig. 7 shows the result of a radio receptor assay for the recombinant IGF-II obtained after chemical clearage of the dual affinity fusion (■) and human IGF-II control, purified from human serum (□). The indicated amounts of standard were used and the recombinant sample was added in 1:10 dilution steps. The relative inhibition of $^{125}$I-IGF-II purified from human serum is shown.

Specific embodiments of the invention will now be described in detail.

## Starting materials

E.coli strains RR1 del M1S (Langley et al., Proc.Natl.Acad-.Sci.USA, 72, 1254-1257 (1975)), HB101 (Maniatis, I. et al., Molecular cloning, a laboratory manual, Cold Spring Harbor Laboratory, Cold Spring, NY) and RV308 (Maurer, R. et al., J.Mol:Biol. 139, 147-161 (1980)) were used in the example. The cloning vehicles used were pEZZ8 (Löwenadler, B. et al., Gene 58, 87-97 (1987)) and pEZZ BIB2. (4p2).

All the strains and vectors are available at the Department of Biochemistry, Royal Institute of Technology, Stockholm, Sweden. The plasmid vector pNP-2 has been deposited with the Deutsche Sammlung von Mikroorganismen (DSM) Braunschweig, Federal Republic of Germany, under No. DSM-4387 on February 3, 1988.

## Buffers and Media

TSB: 30 g Tryptic Soy Broth, made up to 1 litre and autoclaved. TST: TRIS (25mM) and HCl to pH 7.4, 200 mM NaCl, 0.05% Tween 20.

## Routine Methods

methods used routinely in molecular biology are not described (like the use of commercial restriction enzymes, DNA-ligations, Bal 31 exonuclease, S1 nuclease and Klenow polymerase, transformation of E.coli and isolation of plasmid DNA).

The osmotic shock procedure described by Nossal and Heppel, J. of Biol.Chem., vol 244, No. 13, pp 3055-3062 (1966) involves exposal of the cells to 20% sucrose with 0.1 mM EDTA followed by dispersal in cold $5 \cdot 10^{-4}$ M $MgCl_2$ which causes the periplasmic content to be liberated.

In order to analyze protein fractions by SDS-PAGE using the PHAST-system (Pharmacia, Uppsala, Sweden), the samples were dissolved in loading buffer [2.5% SDS, 5% Dithiothreitol (DTT) and 0.01% Bromphenol blue]. Gradient (8-25%) polyacrylamide gels with 5% SDS were run at 10mA for approx. 30 min. and subsequently stained with Coomassie-blue.

The IGF-II radio receptor assay (RRA) was performed as described by Tahano et al (Acta Endocrin.107,164-170,1980).

## EXAMPLE

### Cloning and expression of a fusion protein and the subsequent purification of said protein using both its IgG- and albumin-binding activities.

Based on the amino acid sequence of human native IGF-II, 22 overlapping oligonucleotides, averaging 19 bases in length, were synthesized and the gene was constructed as shown in Fig. 1. Codons recognized by the most abundant tRNAs in E.coli were used although sequences with multiple complementarities were avoided. The 5' and 3' ends of the gene were designed with EcoRI and HindIII cohesive ends, respectively, to facilitate insertion into cloning and expression vectors. A N-terminal methionine codon was included to enable site-specific cleavage of fusion proteins with CNBr to generate native IGF-II. A double TAA translation stop codon was incorporated at the 3'-end of the structural gene.

### Gene synthesis

The oligomers (Fig. 1) were synthesized on an automated machine developed at Kabigen AB using solid phase chemistry and N-protected nucleoside chlorophosphites as described earlier (Nilsson B. et al., Nucl.Acids Res. 13, 1151- 1161 (1985)).

1 nmole deprotected oligomer was kinased by incubation at 37°C for 1 hour with 50 mM Tris-HCL pH 7.6, 10 mM MgCl, 2 nmole ATP, 7 pmole gamma $-^{32}$P-ATP (3000 Ci/nmole) and 10 μ T4 polynucleotide kinase. The phosphorylated oligomers were combined to form three blocks consisting of 6 oligomers and 1 block of 4 oligomers. In all blocks, the oligomers forming the protruding 5'-ends were unphosphorylated to avoid polymerization.

The mixtures were adjusted to 50 mM Tris-Hcl pH 7.6, 10 mM MgCl, and 0.5 mM ATP and incubated for 90°C for 5 minutes followed by slowly cooling to 10°C. The concentration of DTT was adjusted to 10 mM and 10 μ of T4 DNA ligase was added and the mixture was incubated for 20 h at 4°C. The samples were precipitated with ethanol and the ligation product was isolated on 15% polyacrylamide gel (Maniatis, I. et al., Molecular Cloning, a laboratory manual, Cold Spring Harbor Laboratory, Cold Spring Harbor, NY).

The four blocks of DNA were phosphorylated and ligated as described above and the 215 bp fragment was isolated from a 15% polyacrylamide gel. This fragment was ligated into phage M13 mp19 restricted with EcoRI and HindIII and white plaques were analyzed by DNA sequencing (Olsson, A. et al. 45, 175-181 (1986)).

### Expression in E.coli

A secretion vector was constructed by inserting the IGF-II gene into the fusion vector pEZZ between the EcoRI and HindIII sites. The obtained plasmid pRIT19 (Fig. 2) encodes the signal sequence of SPA, two synthetic IgG-binding domains (ZZ), a unique methionine residue followed by the 67 amino acid residues of the major form of human IGF-II.

E.coli strain RV 308 were transformed with the plasmid and the proteins from the expression in overnight shake flasks was analyzed.

The cell cultures were clarified by cross-flow micro filtration and the media were passed through an IgG Fast Flow Sepharose column. Bound material was eluted and lyophilized as described before (Moks, T et al., Bio/Technology 5, 379-382 (1987)).

The purity of the affinity purified and lyophilized material was analyzed by SDS-PAGE, using reducing conditions. As a control, affinity purified human IGF-I fusion protein (Moks, T. et al., Bio/Technology, 5, 379-382 (1987)) with a similar structure and size as IGF-II, was included (Fig. 3, lane 2). The gel reveals that IgG-II (lane 3), although grown at the same conditions as the IGF-I, is highly degraded. Using a radio receptor assay (RRA), no biological activity was found when analyzing the cleavage products from CNBr treatment.

To overcome the problems involved dealing with a heterologously degraded gene product, a novel strategy was used in the production of IGF-II; plasmid pRIT19 was digested with restriction enzymes NotI and HpaII, releasing a fragment containing a truncated IGF-II-gene. A synthetic linker (5'-CGGC-GAAATCTGAAATGG, Kabigen, Stockholm, Sweden) and its complementary sequence was added by ligation. After digestion with EcoRI this fragment was inserted in pZZB1B2 previously digested with EcoRI and BamHI. This construct designated pNP-2 (Fig. 4A) thus encodes a fusion protein consisting of a synthetic IgG-binding region, ZZ, derived from staphylococcal protein A, almost native human insulinlike growth factor II and finally an albumin-binding part of streptococcal protein G. Samples of E.coli cells containing this construct have been deposited at DSM in Braunschweig and given the accession No. DSM-4387.

E.coli HB101 cells containing pNP-2 were cultivated over night at 37°C in 2 litres of TSB supplemented with ampicillin (70mg/l). Proteins localized in the periplasmic space were released using an osmotic shock procedure according to known techniques.

For affinity chromatography, IgG-Sepharose was obtained from Pharmacia (Uppsala, Sweden) and HSA-Sepharose was prepared by coupling purified HSA (Kabi-Vitrum, Stockholm, Sweden) to CNBr-activated Sepharose (Pharmacia, Uppsala, Sweden) as described by Axén et al. (1967 Nature 214, 1302-1304). The HSA- and IgG-Sepharose were packed into columns with about 4 ml of gel. A third column was prepared by mixing 2 ml HSA-Sepharose with 2 ml IgG-Sepharose. All columns were equilibrated with TST-buffer. The periplasmic fraction from E.coli cells harbouring pNP-2 was divided into two equal parts and loaded to the IgG- and HSA-columns, respectively. The columns were thereafter washed with 10 column volumes of TST-buffer and 1 volume of 5mM NH₄AC, pH 5.5, the latter to lower the buffer capacity which enables efficient elution. Elution was made with 0.2 M HAc, pH 3.2, and 0.5 M HAc, pH 2.8, for the IgG- and HSA-columns, respectively, and fractions of 1 ml were collected. The OD at 280 nm measured and the relevant fractions (3 and 4) were collected, pooled and lyophilized.

After lyophilization, the various fractions were dissolved in loading buffer and analyzed by Phast electrophoresis (Pharmacia, Uppsala, Sweden) on 20% polyacrylamide gel with 5% NaDodecylsulphate (SDS-PAGE) as described by the supplier.

For the E.coli strain containing pNP-2, the extracellular proteins, before and after purification were analyzed by SDS-PAGE. As can be seen in Fig. 2 the recombinant fusion protein constitutes more than 10% of the total extracellular proteins (lane 1). Affinity chromatography on either IgG-Sepharose (lane 2) or HSA-Sepharose resulted in high yield of pure recombinant protein with less than 10% degradation. Proteins eluted from the HSA-Sepharose-column were after lyophilization resuspended in TST-buffer and loaded to the IgG-Sepharose column. Eluted proteins from that second affinity purification step were, as well as the flow through, was lyophilized and subjected to SDS-PAGE.

In Fig. 2 (lane 4) it is clearly shown that proteins able to interact with both IgG- and HSA-Sepharose correspond to the expected full-length protein with dual affinity-tails, one on either side of the IGF-II moiety. The destiny of the proteins eluted from the HSA-column lacking the IgG-binding capacity is confirmed when analyzing the flow through from the second column passage (lane 5).

These results shown that it is possible to obtain highly purified fusion protein with this two step affinity procedure.

Chemical cleavage

In order to release free IGF-II, full-length protein obtained from affinity purification on first HSA-Sepharose and later IgG-Sepharose, was subjected to specific cleavage with CNBr. The eluted fractions were lyophilized and dissolved in 70% formic acid. Cyanogen bromide, CNBr, cleaving at the unique Met-residues immediately before and after the IGF-II moiety (Fig. 1B), was added to approximately 200-fold molar excess as calculated on methionine. Cleavage was performed at room temperature for 24 hours, followed by the addition of 9 volumes of water. The solution was freezed and lyophilized. Th cleavage product was dissolved in HAc and diluted with water to 10% and NH₄Ac added to a final concentration of 100mM NH₄Ac. The mixture was desalted on a column of Sephadex G-25 (Pharmacia, Sweden).

The cleaved material before and after cleavage was subjected to analysis on 20 % SDS-PAGE and the results are shown in Fig. 6, lanes 1 and 2. The cleavage yields several new bands (lane 2) corresponding well to the sizes of the albumin-binding fragment (25 kDa), the ZZ-fragment (14 kDa) and the human IGF-II (7 kDa). To confirm this, the cleaved material was passed through a mixed affinity chromatography column containing Sepharose with both IgG and HSA. Material bound as well as not bound (flow through) were analyzed by SDS-PAGE. The results confirm that the 25 kDa fragment and the 14 kDa fragment bind to the mixed column through its albumin- and IgG-binding activity (lane 4), while the low-molecular weight material (7 kDa) is not bound (lane 3).

Biological activity

The flow through material from the mixed affinity column (Fig. 6, lane 3) was analyzed for biological activity in a radio receptor assay using purified human IGF-II as standard. The results presented in Fig. 7 show that the obtained material has activity and that the two curves are parallel in this assay.

This shows that a biologically active human growth hormone can be produced with a dual flanking affinity fusion approach.

**Claims**

1. A recombinant fusion protein comprising a desired protein, oligo- or polypeptide flanked by affinity fragments or moieties, each capable of selective binding.

2. A recombinant fusion protein according to claim 1, wherein said fragments are different.

3. A recombinant fusion protein according to claim 1 or 2, wherein said fragments are selected from:
immunoglobulin-binding, albumin-binding, biotin-binding, thiol-binding, carbohydrate-binding and metallo-binding fragment or moieties.

4. A recombinant fusion protein according to claim 3, wherein said fragments are selected from IgG-binding and albumin-binding moieties.

5. A recombinant fusion protein according to claim 4, wherein a first fragment is IgG-binding and the other fragment is albumin-binding.

6. A recombinant fusion protein according to claim 5, wherein said first fragment is related to the IgG-binding domain of staphylococcal protein A.

7. A recombinant fusion protein according to claim 5 or 6, wherein said other fragment is related to the albumin-binding region of streptococcal protein G.

8. A recombinant vector capable of replication in a host cell, said vector comprising a first DNA sequence coding for a first affinity fragment and, operatively linked thereto, a second DNA sequence coding for a desired protein, oligo- or polypeptide, and further, operatively linked to said second DNA sequence, a third DNA sequence coding for another affinity fragment, said fragments being capable of selective binding.

9. A recombinant vector according to claim 8, wherein said first and third DNA sequences code for different affinity fragments.

10. A recombinant vector according to claim 8 or 9, wherein said first and third DNA sequences code for fragments selected from IgG-binding and albumin-binding moieties.

11. A recombinant vector according to claim 10, wherein said first DNA sequence codes for an IgG-binding fragment, and wherein said third DNA sequence codes for an albumin-binding fragment.

12. A recombinant vector according to claim 11, wherein said first DNA sequence codes for a fragment relating to the IgG-binding domain of staphylococcal protein A.

13. A recombinant vector according to claim 11 or 12, wherein said third DNA sequence codes for a fragment related to the albumin-binding region of streptococcal protein G.

14. A process for purifying and isolating a desired protein, oligo- or polypeptide, comprising the steps:

a) expressing in a recombinant host cell a recombinant fusion protein comprising a desired protein, oligo- or polypeptide flanked by affinity fragments or moieties, said fragments being different;

b) selectively immobilizing said fusion protein by interaction of a first segment with a first ligand attached to a carrier therefor;

c) eluting the immobilized fusion protein, optionally without said first fragment, from said carrier;

d) selectively immobilizing the protein eluted resulting from step c) by interaction of the second fragment with a second ligand attached to a carrier therefor; and

e) eluting the immobilized protein, optionally without said second fragment, from said carrier; and

f) recovering the protein eluted in step e).

15. A process according to claim 14, wherein in steps c) and e) said fusion protein is eluted.

16. A process according to claim 15 comprising the further step of removing said flanking fragments from said fusion protein to form the desired protein, oligo- or polypeptide and recovering same from the mixture.

17. A process according to claim 16, wherein said recoverage is performed by bringing said mixture into contact with a mixed ligand carrier immobilizing said fragments having the desired protein, oligo- or polypeptide free for recoverage.

18. A host organism transformed by the recombinant vector according to any of claims 8 to 13.

19. A host organism according to claim 18, which is a bacterial host, such as a strain of Escherichia, Bacillus or Staphylococcus.

*Fig. 1*

```
                                                                        -5
                                                                        A1
                                                                        AAT TCT
                                                                            GA
                                                                            B1

1        7        13       19       25       31       37       43
MET ALA  TYR ARG  PRO SER  GLU THR  LEU CYS  GLY GLY  GLU LEU  VAL ASP
             A2                          A3
ATG GCT  TAC CGT  CCG TCT  GAA ACC  CTG TGC  GGT GGT  GAA CTG  GTT GAC
TAC CGA  ATG GCA  GGC AGA  CTT TGG  GAC ACG  CCA CCA  CTT GAC  CAA CTG
                      B2                          B3

49       55       61       67       73       79       85       91
THR LEU  GLN PHE  VAL CYS  GLY ASP  ARG GLY  PHE TYR  PHE SER  ARG PRO
    A4                          A5                          A6
ACC CTG  CAG TTC  GTT TGC  GGT GAT  CGT GGT  TTC TAC  TTC TCT  CGT CCA
TGG GAC  GTC AAG  CAA ACG  CCA CTA  GCA CCA  AAG ATG  AAG AGA  GCA GGT
             B4                          B5

97       103      109      115      121      127      133      139
ALA SER  ARG VAL  SER ARG  ARG SER  ARG GLY  ILE VAL  GLU GLU  CYS CYS
             A7                          A8
GCT TCT  CGT GTT  TCT CGT  CGT TCT  CGA GGT  ATC GTT  GAA GAA  TGC TGT
CGA AGA  GCA CAA  AGA GCA  GCA AGA  GCT CCA  TAG CAA  CTT CTT  ACG ACA
B6                          B7                          B8

145      151      157      163      169      175      181      187
PHE ARG  SER CYS  ASP LEU  ALA LEU  LEU GLU  THR TYR  CYS ALA  THR PRO
    A9                          A10                              A11
TTT CGT  TCT TGT  GAC CTG  GCT CTG  CTG GAA  ACC TAC  TGC GCT  ACC CCG
AAA GCA  AGA ACA  CTG GAC  CGA GAC  GAC CTT  TGG ATG  ACG CGA  TGG GGA
             B9                          B10

193      199
ALA LYS  SER GLU

GCG AAA  TCT GAA  TAA TA
CGC TTT  AGA CTT  ATT ATT  CGA
    B11
```

*Fig. 2*

Fig.3

Fig.4

Fig.5

EP 0 333 691 A2

**Fig. 6**

**Fig. 7**